# EUROPEAN PATENT SPECIFICATION

(11) **EP 2 293 073 B1**
(45) Date of publication and mention of the grant of the patent: **31.12.2014**
(21) Application number: 10183043.8
(22) Date of filing: 19.06.2006
(51) Int. Cl.: G01N 33/68, C07K 14/47

(54) **Method for diagnosing neuro-degenerative disease**
Verfahren zur Diagnose neurodegenerativer Erkrankungen
Procédé pour le diagnostic de maladies neuro-dégénératives

(30) Priority: 17.06.2005 GB 0512401
(43) Date of publication of application: 09.03.2011
(62) Divisional of application: 06744252.5
(73) Proprietor: Randox Laboratories Ltd., Crumlin, Co. Antrim Northern Ireland, BT29 4QY (GB)
(72) Inventor: Zellner, Maria, 1090 Vienna (AT); Oehler, Rudolf, 1090 Vienna (AT)
(74) Representative: Jappy, John William Graham

(56) References cited:
- WO-A2-01/72295
- WO-A2-2005/040422
- US-A1- 2003 064 411
- KOELSCH H ET AL: "Polymorphisms in glutathione S-transferase omega-1 and AD, vascular dementia, and stroke", NEUROLOGY, LIPPINCOTT WILLIAMS & WILKINS, PHILADELPHIA, US, vol. 63, no. 12, 28 December 2004 (2004-12-28), pages 2255-2260, XP002405332, ISSN: 0028-3878
- ZELLNER M ET AL: "Quantitative validation of different protein precipitation methods in proteome analysis of blood platelets", ELECTROPHORESIS, WILEY INTERSCIENCE, DE, vol. 26, no. 12, 13 May 2005 (2005-05-13), pages 2481-2489, XP002398363, ISSN: 0173-0835
- MATTILA K M ET AL: "Two-dimensional analysis of qualitative and quantitative changes in blood cell proteins in Alzheimer's disease: search for extraneuronal markers", APPLIED AND THEORETICAL ELECTROPHORESIS : THE OFFICIAL JOURNAL OF THE INTERNATIONAL ELECTROPHORESIS SOCIETY,, vol. 4, no. 4, 1 January 1995 (1995-01-01) , pages 189-196, XP009146243,
- CATTABENI F ET AL: "Platelets provide human tissue to unravel pathogenic mechanisms of Alzheimer disease", PROGRESS IN NEURO-PSYCHOPHARMACOLOGY & BIOLOGICAL PSYCHIATRY, OXFORD, GB, vol. 28, no. 5, 1 August 2004 (2004-08-01) , pages 763-770, XP004560938, ISSN: 0278-5846, DOI: 10.1016/J.PNPBP.2004.05.022

## Description

### Field of the Invention

This invention relates to a method of diagnosing Alzheimer's disease.

### Background to the Invention

Neuro-degenerative diseases are increasing dramatically. Data suggest that neuronal health problems are among the most important contributors to the global burden of disease and disability.

Diagnostic markers for neurological disorders are especially important in diagnosis early in the course of disease, when therapeutic compounds have the greatest potential effect, but accurate diagnosis is difficult. Few diagnostic markers for early stage neuronal disorders are available; those that are available rely on the analysis of a sample material (e.g. cerebrospinal fluid), which is difficult and painful to obtain. Therefore, the characterisation of diagnostic markers which are detectable in the peripheral blood is of great importance. Such peripheral markers would allow preventive screening of a population at risk.

Alzheimer's disease is the major cause of dementia in industrialised countries. In a minority of cases there is a clear family history, with autosomal mutations on the amyloid precursor protein gene. However, the majority of Alzheimer's disease patients have no obvious family history and are classified as sporadic Alzheimer's disease. The aetiology of sporadic Alzheimer's disease is largely unknown (Blennow K and VanMechelen E, J Neural Transm Suppl. 1998; 53:223-35).

Currently, Alzheimer's disease is diagnosed primarily by the exclusion of other known causes of dementia. Diagnosis at an early stage prior to irreversible changes is virtually non-existent. However, pharmaceutical therapy is probably most effective early in the course of disease, before neuro-degeneration is too severe and widespread. Therefore, there is a great need for minimally-invasive diagnostic markers for diagnosis early in the course of the disease.

Cattabeni et al (2004. Progress in Neuro-psychopharmacology & Biological Psychiatry 28(5):763-770) teaches that platelets express and process the amyloid precursor protein (APP) in a manner similar to that seen in the brain.

Published US patent application US 2003/0064411 describes reduced expression of glutathione S-transferase protein identified by SwissProt Accession No. P78417 in brain tissues obtained from patients suffering from Alzheimer's disease.

### Summary of the Invention

The present invention is based on the identification of proteins, isolated from platelets, that can be used as markers for Alzheimer's disease.

According to a first aspect of the invention, a method for the diagnosis of Alzheimer's disease comprises measuring the level of expression of mutant A140D glutathione S-transferase protein identified by SwissProt Accession No. P78417 in a sample of platelets isolated from a person suspected of having Alzheimer's disease, and determining whether the levels of expression is altered compared to a control, wherein a reduced expression level when compared to a healthy non-diseased control of mutant A140D glutathione S-transferase protein identified by SwissProt Accession No. P78417 indicates the presence of a risk of Alzheimer's disease.

The diagnosis involves the isolation of platelets. In a preferred embodiment, the platelets should not be activated during the purification from a blood sample.

According to a second aspect of the invention, use of a kit for the diagnosis of Alzheimer's disease in a person suspected of having Alzheimer's disease in a method according to any of claims 1 to 6, the kit comprising a probe that allows identification of mutant A140D glutathione S-transferase protein and reagents for an immunoassay, 2D gel electrophoresis or polymerase reaction for detecting the level of expression of mutant A140D glutathione S-transferase protein identified by SwissProt Accession No. P78417.

### Description of the Drawings

The Invention is described with reference to the following drawings, wherein:
Figure 1 illustrates cell activation levels for untreated platelets, platelets prepared using chromatography and centrifugation prepared platelets; and
Figure 2 illustrates the technique of Difference Gel Electrophoresis (DIGE).

### Detailed Description of the Invention

This invention provides methods of diagnosing the presence of a risk of Alzheimer's disease based on the detection of the expression level of a specific protein, that is expressed in the platelets of a person suspected of having the disease. The expression level of the protein in the platelets is determined outside of the body (*ex-vivo*) of the person suspected of having the disease, in an *in vitro* assay. The protein that can be detected to diagnose Alzheimer's disease is listed in Table 1. The "Change %" column represents the disease-related increase or decrease (negative number) in expression compared to a non-diseased control.

**Table 1 Alzheimer's Disease Markers**

| **SwissProt Accession No. *** | **Protein name** | **Variant** | **Calcul ated pl** | **MW (kDa)** | **Change % AD/Control** |
|---|---|---|---|---|---|
| P78417 | Glutathione S transferases ω1 | A140D | 5.87 | 30.0 | -32 |

* The SwissProt protein knowledgebase is an annotated protein sequence database established in 1986. It is maintained collaboratively by the Swiss Institute for Bioinformatics (SIB) and the European Bioinformatics Institute (EBI). The Swissprot release referred to herein is v50.1, of 13 June 2006. SwissProt can be accessed at http://expasy.ch and at the SIB and EBI websites, www.isb-sib.ch and www.ebi.ac.uk, respectively. A review of the SwissProt database is provided by Bairoch A et al, Brief Bioinform. 2004 Mar;5(1)39-55.

Table 1 indicates the Swiss Prot accession number for the protein. This identifies the mRNA product that codes for the protein. The specific variant indicated in column 3 of Table 1 is used according to the invention. More preferably, the protein has the pl (where indicated) and molecular weight (MW) indicated in columns 4 and 5 of Table 1. Determining the MW of a protein is a routine experiment for one skilled in the art, for example this can be performed by SDS-PAGE. Determing the pl is also routine to one skilled in the art, using the technique of Isoelectric Focussing. The pl of the protein in Table 1 was determined by the Decyder^{™} 2D analysis software (GE Healthcare, Chalfont St. Giles, UK).

The method requires the detection of the expression level of the specified protein, from platelets of a person, usually a person suspected of having Alzheimer's disease or a person thought to have a predisposition to the disease. As used herein the term "expression level" refers to the amount of the specified protein (or mRNA coding for the protein) in the sampled platelets. The expression level is then compared to the expression level of the protein from platelets of a control. The control may be a platelet sample of a person that is known to have the disease, or a sample from a person that is known not to have the disease. It will be apparent to the skilled person that comparing expression levels of a control and the test sample will allow a decision to be made as to whether the expression level in the test sample and control are similar or different and therefore whether the patient has or is at risk of the disease. In the invention, the expression of the protein from the test sample will be compared to a healthy, i.e. non-diseased, control. The protein may have the same or similar increase or decrease (compared to the control) in expression as compared to the test sample. Whether an increase or decrease of expression is indicative of the presence of disease is indicated in column six of Table 1, where a negative value indicates a decrease in expression.

For the avoidance of doubt, a reduced level when compared to a healthy non-diseased control of glutathione S transferase omega 1 pl 5.87 variant indicates the presence of a risk of Alzheimer's Disease. The control may be a sample taken from a person known to be healthy or known to be diseased (as required) and may be analysed at the same time as the test sample. However, it is preferable that the control is a known value, or level of expression, that is known to be indicative of a healthy (or diseased) person. Once this control level is known, control tests need not be carried out each time a test sample is analysed; the sample can simply be compared to the known control values.

It will be apparent to the skilled person that the full length protein, for example as described in the Swiss Prot database, may be detected. Alternatively, the identification of a fragment (or number of fragments) is possible, provided this allows accurate identification of the full length protein; for example protease digestion of a (full length) protein followed by mass spectrometry of a small number of the resulting peptides is routinely used to identify a protein. This method is often used to identify a spot on a protein gel. If the protein is an oligomer, identification of a single chain may be sufficient.

Methods of measuring the level of expression of a protein from a biological sample are well known in the art and any suitable method may be used. Protein or nucleic acid from the sample may be analysed to determine the expression level. An example of a suitable method is a quantitative PCR reaction on mRNA (or cDNA) obtained from the sample platelets. The use of quantitative PCR to detect gene expression levels is well-known in the art. Kits for quantitative PCR-based gene expression analysis are commercially available, for example the Quantitect system manufactured by Qiagen. Simultaneous analysis of expression levels in multiple samples using a hybridisation-based nucleic acid array system is well known in the art and is also within the scope of the invention.

The level of specific protein (or proteins) in a sample may be detected directly using a number of techniques that will be apparent to one skilled in the art. In summary, suitable techniques typically involve contacting the sample potentially containing a protein from Table 1 with a probe molecule that bids specifically to said protein. A wash step removes unbound molecules. The amount of bound probe molecules is then detected, thereby determining the amount of target molecules in the sample. Preferably, to aid detection, the probe is labelled.

For analysis of a relatively small number of proteins, a quantitative immunoassay such as a Western blot or ELISA can be used to detect the amount of protein (and therefore level of expression) in a sample.

To analyse a larger number of samples simultaneously, a protein array may be used. Protein arrays are well-known in the art and function in a similar way to nucleic acid arrays, primarily using known immobilised proteins (probes) to "capture" a protein of interest, for example a protein identified in Table 1. When the protein is an enzyme, an enzyme assay can be used to analyse the amount of enzyme present in a sample. A protein array contains a plurality of immobilised probe proteins. Preferably, the array contains probe proteins with affinity for different target proteins, allowing a number of different proteins to be analysed on the same support surface, preferably simultaneously. Two or more probe molecules with affinity for a protein identified in Table 1 may be immobilised onto a suitable protein array support material. More preferably, 3, 4, 5 or more different probe molecules may be immobilised, for example 10 or more. The advantages of this multiplexing are faster analysis of multiple samples and the ability to detect multiple markers simultaneously, which allows for combinations of markers to be assayed thereby increasing diagnostic accuracy. A support material suitable for use in a protein array is disclosed in EP-A-0874242. A protein array can be analysed using the Randox Evidence™ System, which is commercially available from Randox Laboratories Limited, Ardmore, Northern Ireland, United Kingdom.

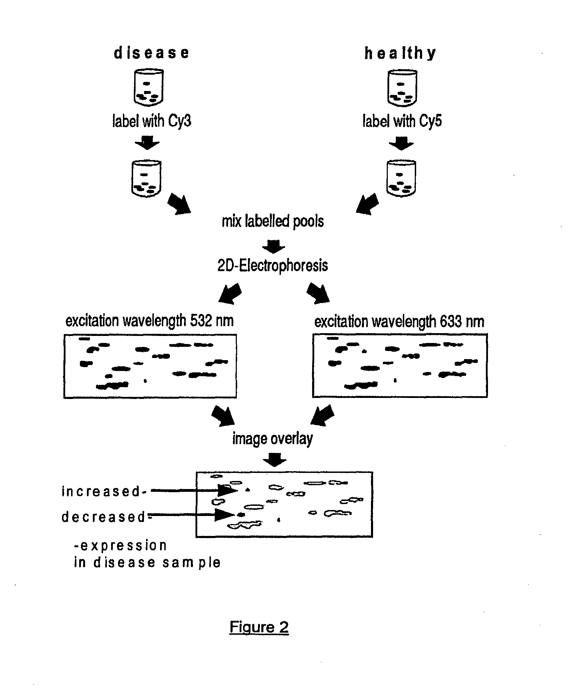

Alternatively, 2D Gel Electrophoresis can be used to analyse simultaneously the expression level of a number of proteins. This method is well known in the art; a sample containing a large number of proteins are typically separated in a first dimension by isoelectric focusing and in a second dimension by size. Each protein resides at a unique location (a "spot") on the resulting gel. The amount of protein in each spot, and therefore the level of expression, can be determined using a number of techniques. An example of a suitable technique is silver-staining the gel followed by scanning with a Bio-rad FX scanner and computer aided analysis using MELANIE 3.0 software (GeneBio). Alternatively, Difference Gel Electrophoresis (DIGE) may be used to quantify the expression level (see Von Eggeling et al; Int. J. Mol Med. 2001 Oct; 8(4):373-7). This technique is summarised in Figure 2.

Blood platelets are non-nucleated cell fragments involved in blood clotting. The platelets may be analysed directly from a whole blood preparation. However, it is preferred that at least some purification is carried out to separate the platelets from other blood components. In one embodiment, a plasma preparation is prepared from a whole blood preparation and expression levels in the platelets detected directly from the plasma. Methods of separating plasma from other blood components are well known in the art, for example, plasma can be prepared from anti-coagulated blood by centrifugation. The platelets may be further purified from the plasma so that a platelet preparation that is substantially free from other blood components is obtained. This usually requires an initial mild centrifugation at approximately 200g, to separate plasma from the red and white cellular blood components, followed by a strong centrifugation at approximately 2000g, to separate the platelets from the plasma. Preferably, the platelets (that have been separated from the plasma) are then lysed and a platelet extract obtained. Methods of lysing cells and obtaining cell-free extracts are well known in the art.

The purification of platelets and platelet extracts from whole blood using conventional methods (involving strong centrifugation) often leads to activation of the platelets. In particular, the strong centrifugation step required to separate plasma from the platelets leads to considerable activation of the platelets. Activation of platelets is a well known phenomenon which results in exocytosis of secretion vesicles and granules ("degranulation") together with a number of other biochemical changes and a change in shape. Activation often leads to exocytosis of proteins of diagnostic interest and, without wishing to be bound by theory, decreases the diagnostic value of the platelets obtained. Assays to detect the level of platelet activation in a sample are known in the art. A prothrombinase or platelet aggregometry assay may be used or expression of activation markers such as CD62-P (P selectin) and platelet factor 4 can be detected to measure platelet activation, as will be apparent to one skilled in the art. The detection of CD62-P by flow cytometry is described in Example 1.

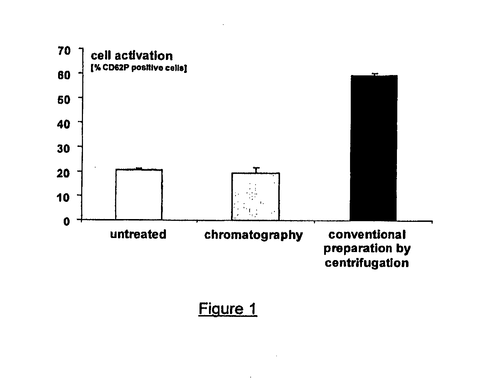

According to the current invention, it is preferred that the platelets from which the level of expression of one or more proteins is measured are non-activated. As used herein, the term "non-activated" refers to platelets that have not been activated by the purification process. Preferably, the platelets are entirely non-activated, although the skilled person will appreciate that platelets that have been slightly activated by the purification process may still be useful. Preferably, the platelet activation level is 50% or less. More preferably, the platelets show 40% or less for example 30% or 20% activation. For the avoidance of doubt, Example 1 and Figure 1 show that platelets prepared according to the current invention show a platelet activation level, as measured by % CD62-P positive cells detected by flow cytometry, of approximately 20%, whereas platelets prepared according to conventional techniques are greater than 60% activated.

A method of isolating non-activated platelet extracts from a whole blood sample may be used in the method according to the claims. It has been found that avoiding strong centrifugation during the purification of the platelets allows non-activated platelets to be obtained. Separating platelets from red and white blood cells using sedimentation or mild centrifugation followed by a separation from plasma by size exclusion filtration (instead of the conventional method requiring strong centrifugation) allows non-activated platelet extracts to be isolated. As used herein, the term "strong centrifugation" refers to the centrifugation forces used typically to pellet platelets for separation from plasma. Strong centrifugation typically requires centrifugation at 1000g or greater, for example 1500g or 2000g. "Mild centrifugation" refers to centrifugation forces that do not cause activation of platelets. In a preferred embodiment, weak centrifugation involves forces of 500g or less, more preferably 250g or less, for example 100g or 50g. In the embodiment where sedimentation is used to separate the red and white blood cells, it is preferred that, prior to sedimentation, the whole blood sample is diluted with an isotonic buffer, which may contain additives to achieve an appropriate density of plasma. For example, (anti-coagulated) blood can be mixed with phosphate buffered saline (PBS) prior to sedimentation. Alternatively, (anti-coagulated) blood can be mixed directly with Dextran 500 (in PBS, e.g. 1.25% Dextran) to a final concentration of between 0.1 % and 5%, preferably 0.25%. Dextran) to a final concentration of between 0.1% and 5%, preferably 0.25%.

Using sedimentation to separate biological samples is well known in the art and any sedimentation method may be used. Preferably, the technique of dextran sedimentation is used, wherein the whole blood sample is applied to a solution containing dextran at a density suitable for separating platelets and plasma from other blood components (see, for example, Bertoluzzo et al, 1999 Blood Cell, Mol. Dis. 26(22):339-349). When left untouched, the plasma and platelets will separate from the other blood components, forming a yellowish supernatant. An advantage of using sedimentation, rather than mild centrifugation, to separate the platelets from red and white blood cells is that it avoids the need for expensive centrifuge equipment.

Sedimentation or mild centrifugation of a whole blood sample results in a supernatant containing platelets and plasma. Although this supernatant may be analysed directly, it is preferred that further purification occurs, to separate the platelets from the plasma. This separation is traditionally carried out by subjecting the platelet suspension (supernatant) to a strong centrifugation step, which leads to a substantial activation of the platelets. It has now been found that avoiding this strong centrifugation step allows the preparation of non-activated platelets. Preferably, the platelets are separated from the supernatant of the sedimentation step by size exclusion filtration, such as size exclusion gel chromatography. The size exclusion gel has a molecular weight exclusion that is capable of fractionating or excluding the platelets to separate them from plasma components. Size exclusion gels with suitable molecular weight cutoffs will be apparent to the skilled person. It is preferred that the molecular weight cutoff is 40 MDa or greater. Typically, the size exclusion gel is packed into a column and the platelet suspension resulting from the sedimentation or mild centrifugation of the whole blood sample (e.g. the supernatant of the sedimentation step) is passed through the column, providing an early column eluate containing very high molecular weight components which have been excluded by the size exclusion gel and a later column eluate containing lower molecular weight components which have been included by the chromatography gel. The platelets will be present in the excluded fraction.

To obtain an extract, the platelets (i.e. the excluded fraction) are contacted with a precipitation agent to lyse the platelets and precipitate the released protein. A separate lysing agent may also be used. Lysing agents are well known in the art and are usually detergents. Any precipitation agent may be used, as are well known in the art. A preferred precipitation agent is ethanol. Trichloroacetic acid (TCA) may alternatively be used as a precipitation agent. Preferably, the early column eluate from the size exclusion step (which contains the platelets) is transferred directly from the column into a container which contains the precipitation solution. Once precipitated, the macromolecules in the platelet extract remain stable for a prolonged period of time, even at room temperature.

After precipitation, the precipitate may be centrifuged to obtain a pellet of precipitated proteins.

The preferred method of providing non-activated platelet extract therefore comprises two major steps, a first sedimentation or mild centrifugation step and a second size exclusion step. The lack of strong centrifugation in these two steps prevents activation of the platelets. Example 1 demonstrates this technique and Figure 1 illustrates its effectiveness in preparing non-activated platelets, compared to the conventional (centrifugation) based techniques.

Once the non-activated platelets have been lysed, providing a non-activated extract, further steps involving centrifugation can be used, for example to obtain a pellet of the precipitated proteins.

The precipitated protein produced by this method is preferably resolubilised in aqueous solution prior to an analysis procedure.

The method of providing non-activated platelets is preferably used to produce platelet extracts containing a protein, or combination of proteins listed in Table 1. An assay for quantifying the amount of any protein identified in Table 1 present in a blood sample therefore comprises obtaining a non-activated platelet extract as described above and determining the amount of the precipitated protein produced, as described above.

The use of a kit comprising the means to perform any aspect of the invention for diagnosis of Alzheimer's Disease is within the scope of the invention according to the claims. In one embodiment, a kit comprises the necessary reagents to allow the expression level of the protein in Table 1 to be detected, and to allow a diagnosis to be made. The kit comprises a probe that allows identification of the protein in Table 1. Preferably, the probe is specific to the protein identified in Table 1. Any probe that specifically identifies the presence of the protein can be used. The skilled person will realise that detecting the amount of a bound probe allows the amount of the target molecule to be determined. Preferably, the probe is labelled with a detectable label. Suitable detectable labels will be apparent to the skilled person and include radioactive labels, chemiluminescent labels and bioluminescent labels. As indicated above, the probes may be immobilised in an array, to allow multiplexing.

In a preferred embodiment, the probe is an antibody molecule. As used herein, the term antibody is to be given its standard meaning in the art, i.e. an immunoglobulin molecule. The skilled person will realise that any type of immunoglobulin molecule or fragment thereof can be used, provided that it retains the ability to bind to the specified target molecule. Preferred antibody fragments include Fab and scFv molecules. Recombinant antibodies and monoclonal antibodies are within the scope of the invention. When the probe is an antibody, a quantitative immunoassay is preferably used to detect the presence of the antibody bound to the target protein from Table 1, and therefore the expression level of that target protein. Immunoassays such as Western Blots or ELISAs, and the reagents required to perform such immunoassays, are well-known in the art. The kit can contain all of the reagents required to perform the immunoassay. In an alternative preferred embodiment, the probe is a nucleic acid that can hybridise to a nucleic acid, preferable mRNA, coding for the protein identified in Table 1. In this embodiment, the kit preferably contains reagents, such as primers and a polymerase enzyme, that are required to perform a polymerase reaction, preferably a quantitative polymerase reaction, which (as described above) can be used to detect the expression level of a protein.

The probe can be an aptamer. As used herein, the term "aptamer" is to be given its standard meaning in the art, i.e. an oligonucleotide that can bind to a target molecule. According to the current invention, the aptamer binds to the protein identified in Table 1.

The expression level of the protein can be determined by 2D gel electrophoresis, or DIGE, as indicated above. In this embodiment, the kit comprises reagents necessary to identify the protein identified in Table 1, using these techniques.

In one embodiment, a kit may be used to perform the method according to the claims utilising preparing non-activated platelets. Preferably, this kit comprises a sedimentation agent, filtration device and precipitation agent. As used herein, the term "sedimentation agent" refers to a reagent that can be used to separate blood plasma from anti-coagulated blood, by sedimentation through the sedimentation agent. A preferred sedimentation agent is dextran (as described above). A "filtration device" refers to a device that separates platelets from plasma. A preferred filtration device is a size exclusion column, most preferably with a Mᵣ cutoff of 40MDa or greater. A "precipitation agent" is defined above.

The invention is further described with reference to the following non-limiting Examples.

### Example 1

(a) Blood samples from normal individuals were obtained and treated with dilution buffer in order to demonstrate the efficacy of removing red and white blood cells in accordance with the present invention.
   Anticoagulated blood (4ml), to be used as a control, was diluted 1:1.25 with an isotonic phosphate buffered saline solution (10mM Na₂HPO₄.NaH₂PO₄, pH=7.4, 135mM NaCl, 2.7 mM KCI, 5mM EDTA) and left untouched for 50 minutes. The upper 100µl of the yellowish supernatant was analysed in a hemocytometer (Coulter MicroDiff 18, Beckmann-Coulter, FL). The supernatant contained 395 x 10³/µl platelets, 0.03 x 10⁶/µl red blood cells, and 3.4 x 10³/µl white blood cells. Before sedimentation the blood contained 187 x 10³/µl platelets, 5.7 x 10⁶/µl red blood cells, and 4.3 x 10³/µl white blood cells. Thus, about 98% of red blood cells and 21% of white blood cells were removed by the sedimentation step. The supernatant contains platelets with a purity of more than 92%. The remaining other cells are red blood cells (7%) and white blood cells (1%), which consist mainly of lymphocytes.
   Removal of only the upper 3/4 of the supernatant improves the reduction of white blood cells in the sedimentation step. A typical supernatant prepared using the upper 3/4 only contains 97.4% platelets, 1.8% red blood cells and 0.8% white blood cells. The cell concentrations in the whole blood sample/supernatant were: red blood cell 5.0 x 10⁶/µl / 2.0 x 10³/µl; white blood cell 4.7 x 10³/µl / 0.9 x 10³/µl; platelets 322 x 10³/µl / 108 x 10³/µl. 99.6% of red blood cells and 80.8% of white blood cells are removed by sedimentation and removal of the upper 3/4 of the supernatant.
(b) The platelet-containing supernatant was then treated with a size exclusion gel in order to show the efficacy of removing plasma proteins in accordance with the present invention, as detailed below.
   Sepharose 2B, an agarose-based gel having a molecular weight fractionation of about 80MD was purchased from Amersham-Pharmacia. About 15ml of the gel was washed with phosphate buffered saline (PBS), degassed, packed in a small column having a diameter of 12cm x 1.4cm, and washed with PBS. Excess buffer was drained from the top of the column and 200µl of the platelet-containing supernatant was applied to the top of the gel without disturbing the surface of the gel. After the 200µl had completely entered the gel bed, 15ml of PBS was added stepwise (1ml) per step) to the gel packed column. Every millilitre of the eluent was collected and divided into 3 parts: one part was used to determine the platelet count using a hemocytometer, the second was used to determine the protein concentration according to the standard Bradford protocol and the third part was used to investigate the proteins by sodium dodecyl sulfate polyacrylamide gel electrophoresis (SDS-PAGE).
   The platelets contained in the supernatant eluted early at about 3ml. The plasma proteins, however, elute much later at about 8ml. The analysis of the proteins by SDS-PAGE revealed that the major plasma protein, albumin, eluted between 8ml and 19ml and was absent in the platelet fractions. These data show that applying platelet-containing supernatant of sedimented blood to size exclusion gel chromatography leads to removal of plasma proteins. It was also noted that the remaining white blood cells in the supernatant were separated from the platelets during the size exclusion chromatography; there appeared to be unspecific binding of white blood cells to sepharose, which retards their elution.
(c) Next, the prepared platelet proteins were analysed by 2-dimensional gel electrophoresis and compared to platelet proteins prepared by centrifugation, as described below.
   The eluate prepared above, which contained the non-activated platelets, was precipitated in ethanol.
   For isolation by centrifugation, peripheral blood was centrifuged at 50g for 15 minutes. The supernatant contained the platelet rich plasma (PRP). In the presence of 150nM PGE1 (prostaglandin E1), PRP was subjected to a second (strong) centrifugation at 1500g for 15 minutes. The platelet pellet was then washed two times in isotonic buffer and precipitated in ethanol.
   The precipitate of each preparation was pelleted by high-speed centrifugation and resolubilised in an aqueous buffer containing 8M urea. The protein pattern of both treatment groups was analysed by 2-dimensional gel electrophoresis according to Gerner at al. J Biol Chem 2000; 275:39018-26.
   Both platelet protein preparations contained several hundreds of proteins. However, the protein preparation from centrifuged platelets contained additional low molecular weight proteins, which were identified as plasma proteins. These results show that the present invention yields a high quality platelet protein preparation, which is suitable for proteomics studies and which is not contaminated by plasma proteins.
(d) Finally, the expression of the activation marker CD62P (p-selectin) in platelets prepared as described above was measured and compared to platelets prepared by centrifugation. Untreated blood was used as a negative control. The degree of platelet activation was determined by expression of the specific activation marker CD62P measured by flow cytometry.
   Gel filtered platelets show the same low CD62P expression levels as platelets in untreated blood, whilst platelets prepared by centrifugation exhibited a strongly increased expression level. Even the single centrifugation step to yield platelet rich plasma (PRP) leads to a small increase in CD62P expression. These data are illustrated in Figure 1, which illustrates that preparation of platelet extracts according to the present invention results in greatly reduced level of platelet activation compared to the conventional purification method.

### Example 2

### 2.1 Selection of patients

### 2.1.1 Healthy control

At least a corresponding number of age- and sex-matched controls, without any evidence of neuropsychiatric disorder or cognitive dysfunction, were selected for this study. Control subjects had a standardised clinical profile based on neurological examinations, laboratory blood and urine analysis and neuropsychological assessment.

### 2.1.2 Evaluation of Alzheimer's Disease patients

A reliable diagnosis of AD is, to date, only possible by a post mortem analysis of the brain. However, there are some parameters which indicate the disease. Therefore it was important to evaluate conscientiously patients which should be enrolled in the study. Early stage dementia patients were investigated. These patients are supposed to suffer from AD as determined by medical, epidemiological, and neuropsychological assessments. For further corroboration isolated blood platelets were taken from these patients and the expression and fragment-pattern of the β-amyloid-precursor protein (APP) was analysed by Western blot. The ratio of the two APP-fragments is known to change in the course of Alzheimer's disease (Di Luca, et al.,1998, Arch Neurol 55:1195-1200). APP-ratios in the early stage dementia patients were indistinguishable from that of healthy controls.

The normal APP-ratios found in the early stage dementia patients can either indicate that patients might suffer from a neuro-degenerative disorderwhich is different from AD or derive from their medical treatment. The patients were treated with an acetlycholinesterase inhibitor, which was recently described to abolish disease-induced changes of APP-ratios in AD. Thus, early stage dementia patients are not suitable for the study.

To find a more promising patient group, late stage dementia patients were investigated. A recently published study showed by post mortem analysis that the late stage dementia patient group consists of 70% AD, 15-20% Lewy body disease, and 2-8% vascular hypoxic dementia (Jellinger, 2001, J Neural Neurosurg Psychiatry. 2001 Nov;71:707-8). In addition these patients receive no acetlycholinesterase inhibitors or other treatments which are known to interfere with platelets. Thus, late stage dementia patients seemed to be the best patient group for the study.

In order to reduce the number of false positives (30%) in the late stage dementia patients, the amyloid precursor protein fragments (APP) ratios of these patients were analysed and compared to the APP ratios of age-and sex-matched control subjects. Visually two groups of APP ratios could be distinguished in both study groups from Western blot analysis of the platelet proteins from all collected samples. Most of the control subjects (n = 13) have high APP ratios. This corresponds to the literature. Eight control subjects had reduced APP ratios.

In 14 of the 21 dementia patients, the ratio between the 130 and the 106-110-kd isoforms was markedly low, corresponding to that described for Alzheimer's disease. The APP ratios of the remaining 7 patients were as high as the ratios of the control subjects, which suggested that these patients would have other forms of dementia. This proportion is similar to the former result of the pathologic study since the excluded seven dementia patients with high APP ratios were equivalent to the 30% non-Aizheimer's dementias.

The optical density of the APP bands of the four groups was then measured and the ratios calculated. The visual selection was confirmed by these measurements. A cut-off level for low APP ratios was set at 1.2. Fourteen of the 21 dementia patient had an APP ratio below this cut off level with a mean value of 1.06 ± 0.21 (n = 14). This group has a high probability of AD and was used in the proteomics assay. 13 of the 21 healthy age-matched controls showed an APP ratio higher than 1.2 with a mean value of 1.44 ± 0.21 (n = 13). These volunteers have a high probability of no AD problems, even in the early stage, and were to be used as a control.

The mean age of dementia group classified for the Alzheimer study group was 82.8 ± 5.8 and the mean age of the control group with high APP ratios was 81.7 ± 6.1.

### 2.2 Sample preparation

### 2.2.1 Choice of sample material

Platelets are non-nucleated blood cells which play an essential role in blood clotting. It is expected that post-transcriptional regulatory mechanisms play an important role in disease-specific changes. Therefore a proteomic approach was adopted and disease-specific changes on the protein level were investigated.

### 2.2.2 Blood sampling

Peripheral venous blood was taken without stasis and coagulation was inhibited by citrate. The platelets were prepared immediately after blood drawing.

### 2.2.3 Preparation of platelets

To avoid any platelet activation and de-granulation the preparation of platelet proteins from human blood was carried out without centrifugation immediately after the blood sample was taken in the clinics. The separation of platelets from other blood components occurred in two steps: (1) removal of red and white blood cells by sedimentation and (2) separation of platelets from other plasma components by size exclusion chromatography.

### 2.2.4 Two-dimensional gel electrophoresis

The platelet protein precipitate was solubilised in sample buffer containing urea, thiourea, and CHAPS and applied in the 1st dimension to isoelectric focussing (24 cm Immobiline Dry strips in a pH range of 4-7 and 6 to 9). This leads to a separation of the proteins according to their pl. The IPG (immobilised pH gradient)-strip was then applied in the 2nd dimension on a 11 % SDS-PA gel and the proteins were separated according to their molecular weight. The protein spots were visualised by silver stain. The gels were scanned using a FX scanner from Bio-Rad and the images were analysed with the MELANIE 3.0 imaging analysis software from GeneBio. With 2-D fluorescence difference gel electrophoresis system (DIGE) the platelet protein samples were labelled with fluorescence dye before electrophoresis and the 2D gels were scanned with a

Typhoon scanner (Amersham Biosciences).

The gel images were highly reproducible. For example: in four parallel gels of the same samples 2463, 2645, 2710, and 2487 protein spots were detected respectively. This indicates a very low coefficient of variation of only 4.7 %. However, some spots were very faint and therefore not suitable for further analysis. Taking into account only clear and distinct spots, 1983 spots (77 %) were found to be present in all four gels. This reproducibility is at a similar level as published by other proteomic research groups (for example O'Neill, et al. 2002, Towards complete analysis of the platelet proteome, Proteomics 2 288-305).

### 2.3 Alzheimer's disease

To determine whether platelets from AD patients have specific modifications in their protein expression profile, inter-individual variations had to be overcome. Therefore a two-step strategy was chosen:
1. Analysis of 2D-gels of pooled samples of AD patients (this levels the inter-individual differences out) in order get an overview of the disease-specific differences and to get a list of candidate spots.
2. Analysis of 2D-gels of each single patient in order to evaluate the statistical significance of the candidate spots.

Using the MELANIE-3.0 software, the 2x2 AD gels were compared and a synthetic AD image was calculated which contained all common spots (1925). The corresponding synthetic control image had 1983 common spots. A comparison of these two synthetic gels revealed that 1501 spots are present in both AD and control samples. The approximately 400 not common spots were all very faint and probably due to methodical variations. All 1501 common spots were introduced into a statistic evaluation. The mean expression level of each single spot in the AD group was calculated and compared with the mean of the healthy control group. In addition the significance level of any difference was calculated by a students t-test.

Using this approach 72 protein spots were found which showed a significant (p<0.05) increase or decrease in the spot intensities of AD samples in comparison to healthy control. In order to evaluate the specificity of these 72 spots the expression level of these spots were analysed in 2D gels of each single AD patient and each single control.

From the 20 single intensity values of each spot per group the mean value was calculated together with the students t-test. Twenty-five spots were found to be still statistically significant (see Table 1).

### 2.3.1 Protein identifications by mass spectrometry from Alzheimer-related protein spots

From this study, twenty-five proteins were identified. The proteins are listed in Table 1.

### 3. Conclusion

The experiments detailed herein indicate that the protein identified in Table 1 is a useful marker for the presence of, or risk of Alzheimer's disease.

## Claims

1. A method for the diagnosis of Alzheimer's disease, comprising measuring the level of expression of mutant A140D glutathione S-transferase protein identified by SwissProt Accession No. P78417 in a sample of platelets isolated from a person suspected of having Alzheimer's disease, and determining whether the level of expression is altered compared to a control, wherein a reduced expression level when compared to a healthy non-diseased control of mutant A140D glutathione S-transferase protein identified by SwissProt Accession No. P78417 indicates the presence of a risk of Alzheimer's disease.

2. The method according to claim 1, wherein detection of mutant A140D glutathione S-transferase protein expression identified by SwissProt Accession No. P78417 is carried out on a plasma sample.

3. The method according to any preceding claim, wherein the platelets are purified to be substantially free from other blood components.

4. The method according to any preceding claim, wherein the platelets are non-activated.

5. The method according to any preceding claim, wherein the level of mutant A140D glutathione S-transferase protein expression identified by SwissProt Accession No. P78417 in the platelets is determined from a platelet protein extract.

6. The method according to claim 5, wherein a platelet protein extract is isolated from a blood sample using a method comprising the steps of:
(i) removing red and white blood cells from the sample by sedimentation or mild centrifugation;
(ii) removing platelets from the resulting sample;
(iii) contacting the platelets obtained in step (ii) with an agent to lyse the platelets and precipitate the platelet protein extract;
(iv) optionally centrifuging the resulting lysate to obtain the precipitated proteins.

7. Use of a kit for the diagnosis of Alzheimer's disease in a person suspected of having Alzheimer's disease in a method according to any of claims 1 to 6, the kit comprising a probe that allows identification of mutant A140D glutathione S-transferase protein and reagents for an immunoassay, 2D gel electrophoresis or polymerase reaction for detecting the level of expression of mutant A140D glutathione S-transferase protein identified by SwissProt Accession No. P78417.

8. The use according to claim 7, wherein the probe binds specifically to mutant A140D glutathione S-transferase protein identified by SwissProt Accession No. P78417.

9. The use according to claim 8, wherein the probe is an antibody or an aptamer.

10. The use according to claim 7, wherein the probe is a nucleic acid that hybridises to a mRNA coding for mutant A140D glutathione S-transferase protein identified by SwissProt Accession No. P78417.

11. The use according to claim 7, wherein the kit comprises dextran, a size exclusion gel, trichloroacetic acid or ethanol and a probe that allows identification of mutant A140D glutathione S-transferase protein identified by SwissProt Accession No. P78417.

## Patentansprüche

1. Ein Verfahren zur Diagnose der Alzheimer-Krankheit, das Folgendes beinhaltet: Messen des Grads der Expression von mutiertem A140D-Glutathion- S-Transferaseprotein, identifiziert durch SwissProt-Zugangsnr. P78417, in einer Probe von Thrombozyten, die aus einer Person isoliert wurden, bei der Verdacht auf das Vorliegen der Alzheimer-Krankheit besteht, und Bestimmen, ob der Grad der Expression im Vergleich mit einer Kontrolle verändert ist, wobei ein reduzierter Expressionsgrad im Vergleich mit einer gesunden, nicht erkrankten Kontrolle von mutiertem A140D-Glutathion- S-Transferaseprotein, identifiziert durch SwissProt-Zugangsnr. P78417, anzeigt, dass ein Risiko für Alzheimer-Krankheit besteht.

2. Das Verfahren gemäß Anspruch 1, wobei der Nachweis der Expression von mutiertem A140D-Glutathion- S-Transferaseprotein, identifiziert durch SwissProt-Zugangsnr. P78417, an einer Plasmaprobe durchgeführt wird.

3. Das Verfahren gemäß einem vorhergehenden Anspruch, wobei die Thrombozyten gereinigt sind, so dass sie im Wesentlichen frei von anderen Blutbestandteilen sind.

4. Das Verfahren gemäß einem vorhergehenden Anspruch, wobei die Thrombozyten nicht aktiviert sind.

5. Das Verfahren gemäß einem vorhergehenden Anspruch, wobei der Grad der Expression von mutiertem A140D-Glutathion- S-Transferaseprotein, identifiziert durch SwissProt-Zugangsnr. P78417, in den Thromboyzten aus einem Thrombozytenproteinextrakt bestimmt wird.

6. Das Verfahren gemäß Anspruch 5, wobei ein Thrombozytenproteinextrakt aus einer Blutprobe unter Verwendung eines Verfahrens isoliert wird, das die folgenden Schritte beinhaltet:
(i) Entfernen der Erythrozyten und Leukozyten mittels Sedimentation oder leichter Zentrifugation aus der Probe;
(ii) Entfernen der Thrombozyten aus der resultierenden Probe;
(iii) In-Kontakt-Bringen der in Schritt (ii) erhaltenen Thrombozyten mit einer Substanz zum Lysieren der Thrombozyten und Ausfällen des Thrombozytenproteinextrakts;
(iv) optional Zentrifugieren des resultierenden Lysats, um die ausgefällten Proteine zu erhalten.

7. Verwendung eines Kits zur Diagnose der Alzheimer-Krankheit bei einer Person, bei der Verdacht auf das Vorliegen der Alzheimer-Krankheit besteht, in einem Verfahren gemäß einem der Ansprüche 1 bis 6, wobei der Kit eine Sonde, die die Identifizierung von mutiertem A140D-Glutathion- S-Transferaseprotein erlaubt, und Reagenzien für einen Immunoassay, 2D-Gelelektrophorese oder Polymerasereaktion zum Nachweisen des Grads der Expression von mutiertem A140D-Glutathion- S-Transferaseprotein, identifiziert durch SwissProt-Zugangsnr. P78417, beinhaltet.

8. Die Verwendung gemäß Anspruch 7, wobei die Sonde spezifisch an mutiertes A140D-Glutathion- S-Transferaseprotein, identifiziert durch SwissProt-Zugangsnr. P78417, bindet.

9. Die Verwendung gemäß Anspruch 8, wobei die Sonde ein Antikörper oder ein Aptamer ist.

10. Die Verwendung gemäß Anspruch 7, wobei die Sonde eine Nucleinsäure ist, die an eine Nucleinsäure hybridisiert, die für ein mutiertes A140D-Glutathion- S-Transferaseprotein, identifiziert durch SwissProt-Zugangsnr. P78417, kodiert.

11. Die Verwendung gemäß Anspruch 7, wobei der Kit Dextran, ein Größenausschlussgel, Trichloressigsäure oder Ethanol und eine Sonde beinhaltet, die die Identifizierung von mutiertem A140D-Glutathion- S-Transferaseprotein, identifiziert durch SwissProt-Zugangsnr. P78417, erlaubt.

## Revendications

1. Procédé de diagnostic de la maladie d'Alzheimer, comprenant mesurer le niveau d'expression de protéine A140D glutathion-S-transférase mutante identifiée par la SwissProt no d'accès P78417, dans un échantillon de plaquettes isolées d'une personne soupçonnée avoir la maladie d'Alzheimer et déterminer si le niveau d'expression est modifié comparé à un témoin, dans lequel un niveau d'expression réduit, lorsque comparé à un témoin sain non malade, de protéine A140D glutathion-S-transférase mutante identifiée par la SwissProt no d'accès P78417, indique la présence d'un risque de maladie d'Alzheimer.

2. Procédé selon la revendication 1, dans lequel la détection de l'expression de protéine A140D glutathion-S-transférase mutante identifiée par la SwissProt no d'accès P78417, est effectuée sur un échantillon de plasma.

3. Procédé selon l'une quelconque des revendications précédentes, dans lequel les plaquettes sont purifiées pour être sensiblement exemptes d'autres composants du sang.

4. Procédé selon l'une quelconque des revendications précédentes, dans lequel les plaquettes sont non activées.

5. Procédé selon l'une quelconque des revendications précédentes, dans lequel le niveau de protéine A140D glutathion-S-transférase mutante identifiée par la SwissProt no d'accès P78417 dans les plaquettes, est déterminé d'un extrait de protéine plaquettaire.

6. Procédé selon la revendication 5, dans lequel un extrait de protéine plaquettaire est isolé d'un échantillon de sang en utilisant un procédé comprenant les étapes consistant à :
(i) retirer les cellules rouges et blanches du sang de l'échantillon par sédimentation ou centrifugation douce;
(ii) retirer les plaquettes de l'échantillon résultant;
(iii) mettre les plaquettes obtenues à l'étape (ii) en contact avec un agent pour lyser les plaquettes et précipiter l'extrait de protéine plaquettaire;
(iv) centrifuger optionnellement le lysat résultant pour obtenir les protéines précipitées.

7. Utilisation d'un kit de diagnostic de la maladie d'Alzheimer chez une personne soupçonnée avoir la maladie d'Alzheimer, dans un procédé selon l'une quelconque des revendications 1 à 6, le kit comprenant une sonde qui permet l'identification de protéine A140D glutathion-S-transférase mutante et des réactifs pour un essai immunologique, une électrophorèse sur gel 2D ou une réaction de polymérisation, afin de détecter le niveau d'expression de protéine A140D glutathion-S-transférase mutante identifiée par la SwissProt no d'accès P78417.

8. Utilisation selon la revendication 7, dans laquelle la sonde se lie spécifiquement à la protéine A140D glutathion-S-transférase mutante identifiée par la SwissProt no d'accès P78417.

9. Utilisation selon la revendication 8, dans laquelle la sonde est un anticorps ou un aptamère.

10. Utilisation selon la revendication 7, dans laquelle la sonde est un acide nucléique qui s'hybride à un ARNm codant pour la protéine A140D glutathion-S-transférase mutante identifiée par la SwissProt no d'accès P78417.

11. Utilisation selon la revendication 7, dans laquelle le kit comprend du dextrane, un gel d'exclusion stérique, de l'acide trichloroacétique ou de l'éthanol et une sonde qui permet l'identification de la protéine A140D glutathion-S-transférase mutante identifiée par la SwissProt no d'accès P78417.
